# EUROPEAN PATENT SPECIFICATION

(11) **EP 1 219 619 B1**
(45) Date of publication and mention of the grant of the patent: **11.08.2004**
(21) Application number: 00964689.4
(22) Date of filing: 05.10.2000
(51) Int. Cl.: C07D 405/12, C07D 405/14, C07D 413/14, C07D 409/14, C07D 493/04, A61K 31/443, A61K 31/454, A61K 31/4535, A61K 31/4545, A61P 11/06, A61P 43/00, A61P 1/08

(54) **3-AMINO-2-PHENYLPIPERIDINE DERIVATIVES AS SUBSTANCE P ANTAGONISTS**
3-AMINO-2-PHENYLPIPERIDINDERIVATE ALS SUBSTANZ P ANTAGONISTEN
DERIVES DE 3-AMINO-2-PHENYLPIPERIDINE COMME D'ANTAGONISTES DE LA SUBSTANCE P

(30) Priority: 07.10.1999 JP 28628399
(43) Date of publication of application: 03.07.2002
(73) Proprietor: HISAMITSU PHARMACEUTICAL CO. INC., Tosu-shi, Saga-ken 841-0017 (JP)
(72) Inventor: BEPPU, Kouichi, c/o Hisamitsu Pharma. Co., Inc., Tosu-shi, Saga 841-0017 (JP); MANAKO, Takafumi, c/o Hisamitsu Pharma. Co., Inc., Tosu-shi, Saga 841-0017 (JP); SAKAI, Michinori, c/o Hisamitsu Pharma. Co. Ltd., Tsukuba-shi, Ibaraki 305-08506 (JP); SAEKI, Masakazu, c/o Hisamitsu Pharma. Co. Inc., Tsukuba-shi, Ibaraki 305-0856 (JP); MIZUMA, Hideyuki, Hisamitsu Pharma. Co. Inc., Tsukuba-shi, Ibaraki 305-0856 (JP)
(74) Representative: Westendorp, Michael, Dr.
(86) International application number: PCT/JP2000/006950
(87) International publication number: WO 2001/025233

(56) References cited:
- EP-A1- 0 826 684
- WO-A-99/25714
- WO-A1-95/06645

## Description

### Technical Field

The present invention relates to new piperidine derivatives or pharmaceutically acceptable acid addition salts thereof which provide an antagonistic action against the peptidic neurotransmitter, substance P, and to substance P antagonists having the above derivatives or salts as an effective component. The piperidine derivatives or pharmaceutically acceptable acid addition salts thereof of this invention are useful as prophylactics/remedies for respiratory diseases, central nervous diseases, gastrointestinal diseases, circulatory diseases and various kinds of inflammation and pain.

### Background Art

Substance P is tachykinin undecapeptide found particularly in the mammal and is referred to as neurokinin together with neurokinin A and neurokinin B. It is well known that substance P takes part in various pathological areas including, for example, respiratory diseases such as asthma, central nervous diseases such as anxiety and schizophrenia, gastrointestinal diseases such as ulcerative colitis, circulatory diseases such as hypertension, and inflammation and pain (Journal of Medicinal Chemistry, 25, 1009 (1982), Trends in Cluster Headache, 85-97 (1987), ELSEVIER). On the other hand, there have been known as non-peptidic substance P antagonists compounds having a quinuclidine skeleton (National Publication of International Patent Application No. 3-503768), compounds having a piperidine skeleton (Japanese Patent Laid-Open No. 4-103570), compounds having an isoindolone skeleton (Japanese Patent Laid-Open No. 3-176469), and compounds having an arylalkyl amine skeleton (Japanese Patent Laid-Open No. 3-206086).

There have been reported in International Publication WO 94/13663 Specification, WO 95/06645 Specification and WO 96/30367 Specification aminopiperidines having an oxygen-containing condensed heterocyclic ring such as benzofuran ring or benzopyran ring. There have been, however, neither disclosure of compounds having an oxygen-containing condensed heterocyclic ring with a phenyl group or heterocyclic group attached thereon as well as tricyclic compounds, which are the compounds of this invention, nor description suggesting such compounds.

EP 0 826 684 A1 discloses piperidine derivatives having an antagonism on substance P. The compounds are useful as a preventive or remedy for asthma, vomiting, etc.. The piperidine derivatives comprise a benzyl group substituted by a group R representing hydrogen, halogen, lower alkyl, hydroxy, lower alkoxy, lower alkylthio, lower alkylsulfinyl, lower alkylsulfonyl, substituted aminosulfonyl or nitro.

WO 95/06645 discloses benzofuran derivatives which may be used in the treatment of conditions mediated by tachykinins. The compound comprises a benzofuran group substituted by a phenyl group or a 5- or 6-member aromatic heterocycle containing 1, 2, 3, or 4 heteroatoms selected from oxygen, nitrogen or sulphur. These groups may be bound to the phenol ring via a single bond or a methylene group.

WO 99/25714 discloses piperidinylaminomethyl trifluoromethyl cyclic ether compounds which may act as substance P antagonists. The compounds comprise a benzyl group which is fused to a 5- or 6-membered ring comprising an oxygen atom. To this ring is bound a trifluoromethyl group in a position neighboured to the benzyl ring.

### Disclosure of the Invention

Accordingly, the object of this invention is to provide piperidine derivatives or pharmaceutically acceptable acid addition salts thereof which provide a non-peptidic antagonistic action against substance P and substance P antagonists having the above derivatives or salts as an effective component.

This invention provides piperidine derivatives having the following general formula [I] : 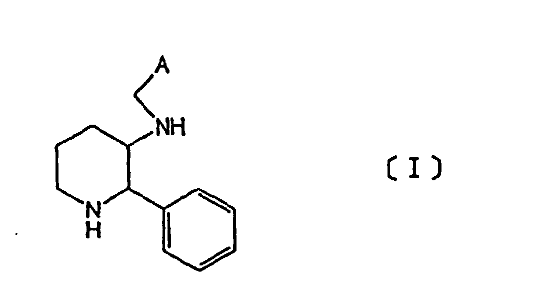 wherein A represents the following formula [A]: 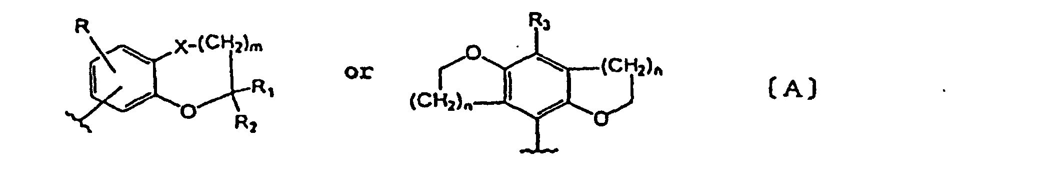 wherein
m is 0 or 1,
n is 1 or 2,
X represents CH₂, O or CH-CH₃,
R represents an unsubstituted or substituted phenyl group or an unsubstituted or substituted heterocyclic group,
R₁ and R₂ may be the same or different and represent a hydrogen atom or a lower alkyl group, respectively, and
R₃ represents a hydrogen atom or a lower alkyl group,
or pharmaceutically acceptable acid addition salts thereof.

This invention also provides cis-piperidine derivatives having the following general formula [Ia]: 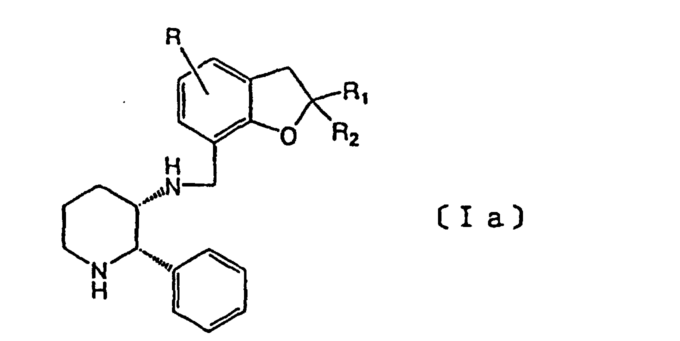 wherein
R represents an unsubstituted or substituted phenyl group or an unsubstituted or substituted heterooyclic group,
R₁ and R₂ may be the same or different and represent a hydrogen atom or a lower alkyl group, respectively,
or pharmaceutically acceptable acid addition salts thereof.

This invention provides cis-piperidine derivatives having the following general formula [Ib]: 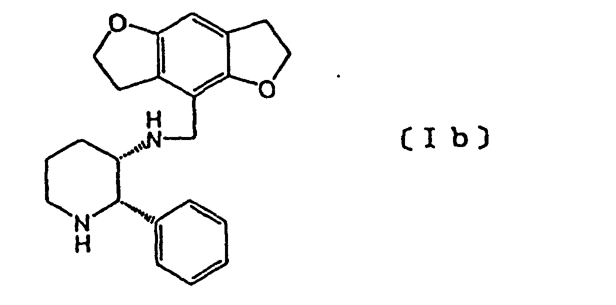 or pharmaceutically acceptable acid addition salts thereof.

This invention provides substance P antagonists having, as an effective component, piperidine derivatives having the above general formula [I] or pharmaceutically acceptable acid addition salts thereof.

This invention provides substance P antagonists having, as an effective component, cis-piperidine derivatives having the above general formula [Ia] or [Ib] or pharmaceutically acceptable acid addition salts thereof.

In the following the invention will be described in further detail.

In the above general formula [I], A has the following formula [A]: 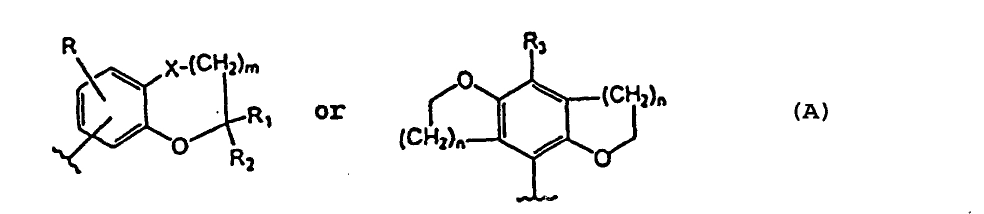 wherein m is 0 or 1, and n is 1 or 2. A takes the form of a condensed ring of a benzene ring and a pentacyclic ring when m=0 and n=1 and of a condensed ring of a benzene ring and a hexacyclic ring when m=1 and n=2.

X represents CH₂, O, or CH-CH₃ which is a methyl-substituted endocyclic methylene group.

R represents an unsubstituted or substituted phenyl group or an unsubstituted or substituted heterocyclic group. The substituted phenyl group has 1 to 4 substituents of the same or different kinds and the substituted heterocyclic group has 1 to 4 substituents of the same or different kinds. The substituent herein mentioned is a halogen atom, a lower alkyl group, a lower alkoxy group or a lower alkylthio group.

Preferably the halogen atom is a fluorine, chlorine bromine or iodine atom.

The lower alkyl group is a cyclic or noncyclic alkyl group with 1 to 6 carbon atoms (which may contain 1 to 5 halogen atoms such as fluorine atoms in arbitrary positions), such as methyl, ethyl, n-propyl, iso-propyl, n-butyl, iso-butyl, tert-butyl, n-pentyl, n-hexyl, cyclopentyl, cyclohexyl, monofluoromethyl, difluoromethyl, trifluoromethyl, trifluoroethyl or perfluoroethyl group.

The lower alkoxy group is a cyclic or noncyclic alkoxy group with 1 to 6 carbon atoms (which may contain 1 to 5 halogen atoms such as fluorine atoms in arbitrary positions), such as methoxy, ethoxy, n-propoxy, iso-propoxy, n-butoxy, iso-butoxy, tert-butoxy, n-pentyloxy, n-hexyloxy, cyclopentyloxy, cyclohexyloxy, monofluoromethoxy, difluoromethoxy, trifluoromethoxy, trifluoroethoxy or perfluoroethoxy group.

Preferably the lower alkylthio group is a cyclic or noncyclic alkylthio group with 1 to 6 carbon atoms (which may contain 1 to 5 halogen atoms such as fluorine atoms in arbitrary positions), such as methylthio, ethylthio, n-propylthio, iso-propylthio, n-butylthio, iso-butylthio, tert-butylthio, n-pentylthion, n-hexylthio, cyclopentylthio, cyclohexylthio, monofluoromethylthio, difluoromethylthio, trifluoromethylthio, trifluoroethylthio or perfluoroethylthio group.

The heterocyclic group is a five-membered or six-membered heterocyclic group containing 1 to 4 heteroatoms selected from the group consisting of oxygen, nitrogen and sulfur atoms, such as pyrrolyl, thienyl, furyl, pyridyl, pyrimidinyl, pyrazolyl, pyrazinyl, pyridazinyl, imidazolyl, oxazolyl, isooxazolyl, thiazolyl, isothiazolyl, 1,2,3-triazolyl, 1,2,4-triazolyl, 1,2,4-oxadiazolyl, 1,3,4-oxadiazolyl or tetrazolyl group.

R₁ and R₂ may be the same or different and each of them represents a hydrogen atom or a lower alkyl group such as methyl, ethyl, n-propyl, iso-propyl, n-butyl or iso-butyl group. The combination of R₁ and R₂ allows A to take the form of an unsubstituted, monoalkyl or dialkyl group.

R₃ represents a hydrogen atom or a lower alkyl group such as methyl, ethyl, n-propyl, iso-propyl, n-butyl or iso-butyl group.

This invention embraces any forms of stereoisomer, diastereoisomer and the mixture thereof which are produced in monoalkyl group. This invention also embraces any of cis-and trans-forms of the compound [I], and the cis-form represented by the compound [Ia] or [Ib] is particularly preferable.

The pharmaceutically acceptable acid addition salts include, for example, salts of the piperidine derivatives and inorganic acid, such as hydrochloric acid, sulfuric acid, nitric acid or phosphoric acid, or of the piperidine derivatives and organic acid, such as acetic acid, propionic acid, glycol acid, lactic acid, malic acid, tartaric acid, citric acid, ascorbic acid, maleic acid, oxalic acid, fumaric acid, succinic acid, malonic acid, methanesulfonic acid, benzenesulfonic acid or toluensulfonic acid; however, the above examples are shown for illustrative purposes only and are not intended to limit this invention.

Preferable compounds of this invention include, for example,
(2S,3S)-3-[(5-(3-methoxyphenyl)-2,3-dihydrobenzofuran-7-yl)methyl]amino-2-phenylpiperidine hydrochloride,
(2S, 3S)-3-[(5-(5-methyl-1,2,4-oxadiazol-3-yl)-2,3-dihydrobenzofuran-7-yl)methyl]amino-2-phenylpiperidine hydrochloride,
(2S, 3S)-3-[(5-(tetrazole-1-yl)-2,3-dihydrobenzofuran-7-yl)methyl]amino-2-phenylpiperidine hydrochloride,
(2S, 3S)-3-[(5-(pyrrole-1-yl)-2,3-dihydrobenzofuran-7-yl)methyl]amino-2-phenylpiperidine hydrochloride,
(2S,3S)-3-[(5-(thiophene-2-yl)-2,3-dihydrobenzofuran-7-yl)methyl]amino-2-phenylpiperidine hydrochloride,
(2S,3S)-3-[(5-(thiophene-3-yl)-2,3-dihydrobenzofuran-7-yl)methyl]amino-2-phenylpiperidine hydrochloride,
(2S,3S)-3-[(5-(pyridine-3-yl)-2,3-dihydrobenzofuran-7-yl)methyl]amino-2-phenylpiperidine hydrochloride,
(2S,3S)-3-[(5-phenyl-2,3-dihydrobenzofuran-7-yl)methyl]amino-2-phenylpiperidine hydrochloride,
(2S,3S)-3-[(5-(2-trifluoromethylphenyl)-2,3-dihydrobenzofuran-7-yl)methyl]amino-2-phenylpiperidine hydrochloride,
(2S,3S)-3-[(5-(3-trifluoromethylphenyl)-2,3-dihydrobenzofuran-7-yl)methyl]amino-2-phenylpiperidine hydrochloride,
(2S,3S)-3-[(5-(4-trifluoromethylphenyl)-2,3-dihydrobenzofuran-7-yl)methyl]amino-2-phenylpiperidine hydrochloride,
(2S, 3S)-3-[(5-(3,5-bis(trifluoromethyl)phenyl)-2,3-dihydrobenzofuran-7-yl)methyl]amino-2-phenylpiperidine hydrochloride,
(2S,3S)-3-[(5-(4-methoxyphenyl)-2,3-dihydrobenzofuran-7-yl)methyl]amino-2-phenylpiperidine hydrochloride,
(2S,3S)-3-[(5-(5-trifluoromethyl-1,2,4-oxadiazole-3-yl)-2,3-dihydrobenzofuran-7-yl)methyl]amino-2-phenylpiperidine hydrochloride, and
(2S,3S)-3-[(2,3,6,7-tetrahydrobenzo[1,2-b:4,5-b']difuran-4-yl)methyl]amino-2-phenylpiperidine hydrochloride.

In the use of compounds having the general formula [I], [Ia] or [Ib] or the pharmaceutically acceptable acid addition salts thereof as a drug, they can be administered as they are; however, they can also be administered together with a known excipient in a proper dosage form such as tablet, capsule, powder, fine granule, suppository, injectable, cream or aerosol. The method of administration is not limited to any specific one; normally, they can be stably administered systemically or topically and orally or parenterally. The dose of compounds having the general formula [I], [Ia] or [Ib] or of the pharmaceutically acceptable acid addition salts thereof is determined properly depending on the symptom, age, sex, body weight, etc. of the subject of the administration. When they are given orally to adults, the preferable dose is normally about 1 mg to 500 mg one to several times daily.

The compounds having the general formula [I], [Ia] or [Ib] of this invention or the pharmaceutically acceptable acid addition salts thereof are useful as an effective component of substance P antagonists. Accordingly, they can be used as prophylactics/remedies for asthma, vomiting, etc.

### (Production Process)

In the following the processes of producing the compounds of this invention and the acid addition salts thereof will be described. The compounds of this invention and the acid addition salts thereof can be produced in accordance with the processes described in International Publication WO 96/30367 Specification as shown below; however, these processes are shown for illustrative purposes only and are not intended to limit this invention. (Step 1)

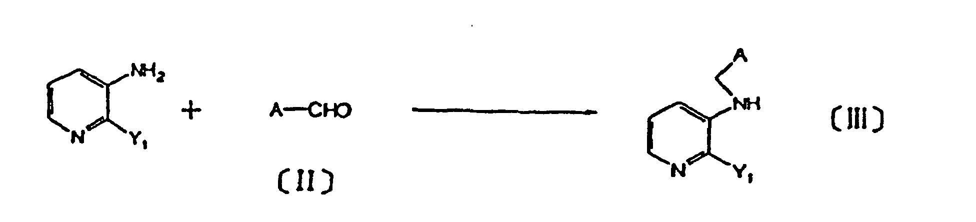

### (Step 2)

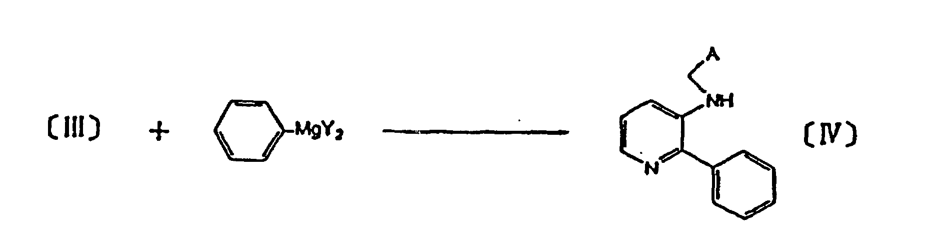

### (Step 3)

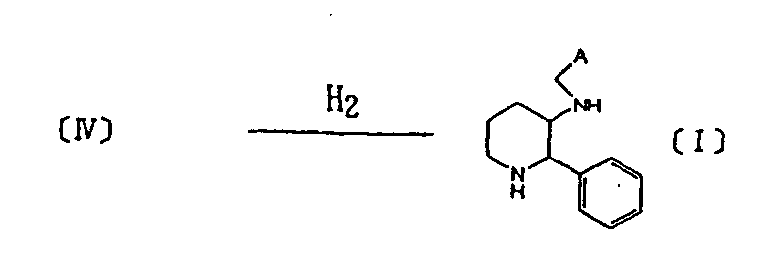

wherein Y₁ represents a halogen atom, a mesyl group or a tosyl group, Y₂ represents a halogen atom, and A is just defined as above.

As shown above, first 3-amino-2-substituted pyridine and formyl compound [II] are reacted with each other in an inert solvent such as lower alcohol (for example, methanol and ethanol) or acetic acid at -20°C to 50°C in the presence of a reducing agent such as sodium cyano borohydride, sodium triacetoxy borohydride or formic acid, so as to obtain the compound [III] without isolating imine as an intermediate (Step 1).

The compound [III] thus obtained and Grignard reagent are subjected to cross-coupling reaction in an inert solvent such as tetrahydrofuran or ether at 0°C to 70°C in the presence of a transition metal catalyst such as {1,3-bis(diphenylphosphino)propane}nickel(II) chloride or {1,2-bis(diphenylphosphino)ethane}nickel(II) chloride, so as to obtain the compound [IV] (Step 2).

Then the compound [IV] thus obtained is subjected to hydrogenation reaction in an inert solvent such as lower alcohol (for example, methanol and ethanol) or acetic acid at 1 to 5 atm in the presence of a metal catalyst such as palladium carbon, platinum oxide or Raney nickel, so as to obtain the compound [I] (Step 3).

Lastly, the racemic mixture [I] is optically resolved so as to obtain the compounds of this invention having a general formula [Ia] or [Ib].

Any acid addition salt of the compound can be formed from a free base. Specifically, a diastereomer salt of (+) isomer with a high optical purity can be produced by mixing the compound [I] and an optically resolving agent such as (2R,3R)-(-)-di-0-benzoyltartaric acid or (R)-(-)-mandelic acid in an inert solvent such as methanol, ethanol or isopropanol and then recrystallizing the produced dibenzoyltartrate or mandelate. Then the recrystallized salt is distributed between an organic solvent such as ether, chloroform or dichloromethane and an aqueous inorganic base such as sodium hydrogencarbonate, sodium carbonate or sodium hydroxide, so as to obtain a compound [Ia] or [Ib], which is an (+) enantiomer, as a free base. And a hydrochloride can be obtained by adding, for example, hydrogen chloride- containing ether to the free base.

### Best Mode for Carrying out the Invention

In the following this invention will be described in further detail while taking examples; however, it is to be understood that these examples are shown for illustrative purposes only and are not intended to limit this invention.

### (Example 1)

8.2 g of 3-[(5-(3-methoxyphenyl)-2,3-dihydrobenzofuran -7-yl)methyl]amino-2-phenylpyridine was dissolved in 50 ml of acetic acid, 0.5 g of platinum oxide was added, and hydrogenation reaction was carried out at 3 atm for 8.5 hours. After completion of the reaction, the catalyst was filtered and the solvent was concentrated. After pieces of ice were added to the residue, the residue was alkalized with 50% sodium hydroxide aqueous solution and extracted with dichloromethane three times. After dried, the extract was distilled and purified by silica gel chromatography (chloroform/methanol/aqueous ammonia = 15 : 1 : 0.05), to obtain 4.3 g of oil-like ais-3-[(5-(3-methoxyphenyl)-2,3-dihydrobenzofuran-7-yl)methyl]amino-2-phenylpiperidine.

### (Example 2)

4.14 g of cis-3-[(5-(3-methoxyphenyl)-2,3-dihydrobenzofuran-7-yl)methyl]amino-2-phenylpiperidine having been synthesized in Example 1 was dissolved in 15 ml of methanol, about 15 ml of methanol solution containing 1.53 g of (R)-(-)-mandelic acid was added, followed by stirring at room temperature for 10 minutes. The residue obtained after concentrating the methanol was crystallized with ether and the crystal was obtained by filtration. The crystal was washed with a small amount of isopropanol and then with ether, to obtain 3.4 g of crude crystal. The obtained crude diastereomer salt was recrystallized from the isopropanol twice, distributed between 1 M sodium hydroxide aqueous solution and dichloromethane, and extracted with 10 ml of dichloromethane under alkaline conditions three times. After drying the organic layer, the extract was concentrated and dried to solid, to obtain an oil-like free base. 3 ml of dichloromethane was added to the oil-like base, ether containing hydrogen chloride was added dropwise, the crystal formed was taken by filtration, the crystal was recrystallized with isopropanol/methanol, to obtain 1.95 g of (2S,3S)-3-[(5-(3-methoxyphenyl)-2,3-dihydrobenzofuran-7-yl)methyl]amino-2-phenylpiperidine hydrochloride. m.p.: 202 to 207°C (dec.)
NMR(DMSO-d₆) : 1.77-1.87 (1H, m), 2.03-2.45 (3H, m), 3.13-3.24 (1H, m ), 3.20 (2H, t, J=8.6Hz), 3.40 (1H, d, J=13.5Hz), 3.43-3.52 (1H, m), 3. 83 (3 H, s), 3.85 (1H, d, J=13.5Hz), 3.97 (1H, m), 4. 53 (2H, t, J=8. 6Hz), 4. 96 (1H, m), 6.84-6.89 (1H, m), 7.15-7.21 (2H, m), 7.29-7.35 (1H, m), 7.41-7.60 (5H, m), 7. 72-7.77 (2H, m), 9. 40 (2H, br), 10. 34 (2H, br)
Values of elemental analysis (C₂₇H₃₀N₂O₂·2HCl)
Calculated value C: 66.53 H: 6.62 N: 5.75
Measured value C: 66.46 H: 6.61 N: 5.79

The following compounds were synthesized in accordance with the processes of the Examples 1 and 2.

### (Example 3)

(2S,3S)-3-[(5-(tetrazole-1-yl)-2,3-dihydrobenzofuran-7-yl)methyl]amino-2-phenylpiperidine hydrochloride
m.p.: 213 to 220°C (dec.)
NMR(DMSO-d₆) : 1.75-1.87 (1H, m), 2.00-2.43 (3H, m), 3.10-3.24 (1H, m), 3.26 (2H, t, J=8. 8Hz), 3.45 (1H, d, J=13.6Hz), 3.42-3.48 (1H, m), 3.86 (1H, d, J=13.6Hz), 3.92 (1H, m), 4.58-4.67 (2H, m), 4.89 (1H, m), 7.37-7,52 (3H, m ), 7.65-7.78 (4H, m), 9.50 (2H, br), 9.89 (1H, s), 10.40 (2H, br)
Values of elemental analysis (C₂₁H₂₄N₆O·2HCl)
Calculated value C: 56.13 H: 5.83 N: 18.70
Measured value C: 56.01 H: 5.79 N: 18.83

### (Example 4)

(2S, 3S)-3-[(5-(5-methyl-1,2,4-oxadiazol-3-yl)-2,3-dihydrobenzofuran-7-yl)methyl]amino-2-phenylpiperidine hydrochloride
m.p.: 200 to 205°C (dec.)
NMR(DMSO-d₆) : 1.76-1.87 (1H, m), 2.03-2.41 (3H, m), 2.63 (3H, s), 3.10 -3.22 (1H, m), 3.23 (2H, t, J=8.5Hz), 3.42-3.48 (1H, m), 3.50 (1H, d, J=13.4H z), 3.84 (1H, d, J=13.4Hz), 3.90 (1H, m), 4.53-4.66 (2H, m), 4.93 (1H, m), 7.4 2-7.55 (3H, m), 7.68-7.83 (4H, m), 9.20 (2H, br), 10.34 (2H, br)
Values of elemental analysis (C₂₃H₂₆N₄O₂·2HCl)
Calculated value C: 59.61 H: 6.09 N: 12.09
Measured value C: 59.49 H: 6.11 N: 11.97

### (Example 5)

(2S,3S)-3-[(5-(pyrrole-1-yl)-2,3-dihydrobenzofuran-7-yl)methyl]amino-2-phenylpiperidine hydrochloride
m.p.: 211 to 221°C (dec.)
NMR(DMSO-d₆) : 1.75-1.87 (1H, m), 2.02-2.44 (3H, m), 3.12-3.24 (1H, m) , 3.19 (2H, t, J=8. 6Hz), 3.33 (1H, d, J=13.6Hz), 3.45-3.51 (1H, m), 3.83 (1H , d, J=13.6Hz), 3. 92 (1H, m), 4.51 (2H, t, J=8.6Hz), 4. 94 (1H, m), 6.21 (2H, t, J=2.1Hz), 7.20 (2H, t, J=2.1Hz), 7.38-7.53 (5H, m), 7.68-7.74 (2H, m), 9.30 (2H, br), 10.19 (2H, br)
Values of elemental analysis (C₂₄H₂₇N₃O·2HCl)
Calculated value C: 64.57 H: 6.55 N: 9.41
Measured value C: 64.45 H: 6.59 N: 9.30

### (Example 6)

(2S,3S)-3-[(5-(thiophene-2-yl)-2,3-dihydrobenzofuran-7-yl)methyl]amino-2-phenylpiperidine hydrochloride
m.p.: 189 to 193°C (dec.)
NMR(DMSO-d₆) : 1.76-1.87 (1H, m), 2.03-2.43 (3H, m), 3.12-3.22 (1H, m) , 3.19 (2H, t, J=8. 7Hz), 3. 43-3. 52 (1H, m), 3. 43 (1H, d, J=13.2Hz), 3. 82 (1H , d, J= 13.2Hz), 3.94 (1H, m), 4.52 (2H, t, J=8.7Hz), 4.94 (1H, m), 7.09 (1H, d d, J=3.78Hz, 5.13Hz), 7.35 (1H, dd, J=1.08Hz, 3.78Hz), 7.43 (1H, dd, J=1.0 8Hz, 5.13Hz), 7.42-7.57 (5H, m), 7.71-7.76 (2H, m), 9. 20 (2H, br), 10.32 (2H ,br)
Values of elemental analysis (C₂₄H₂₆N₂OS·2HCl)
Calculated value C: 62.20 H: 6.09 N: 6.04
Measured value C: 62.05 H: 6.11 N: 6.10

### (Example 7)

(2S,3S)-3-[(5-(thiophene-3-yl)-2,3-dihydrobenzofuran-7-yl)methyl]amino-2-phenylpiperidine hydrochloride
m.p.: 238 to 248°C (dec.)
NMR (DMSO-d₆) : 1.76-1.87 (1H, m), 2.03-2.44 (3H, m), 3.12-3.24 (1H,m) , 3.18 (2H, t, J=8.7Hz), 3.39 (1H, d,J=13.6Hz), 3. 44-3. 52 (1H, m), 3.83 (1H , d, J=13.6Hz), 3.97 (1H, m), 4.51 (2H, t, J=8.7Hz), 4.96 (1H, m), 7.41-7.56( 5H, m), 7.59 (1H, dd, J=3.0Hz, 5.1Hz), 7.65-7.68(2H, m), 7.71-7.76 (2H, m), 9.30 (2H, br), 10.33 (2H,br)
Values of elemental analysis (C₂₄H₂₆N₂OS·2HCl)
Calculated value C: 62.20 H: 6.09 N: 6.04
Measured value C: 62.12 H: 6.07 N: 5.98

### (Example 8)

(2S,3S)-3-[(5-(pyridine-3-yl)-2,3-dihydrobenzofuran-7-yl)methyl]amino-2-phenylpiperidine hydrochloride
m.p.: 187 to 201°C (dec.)
NMR (DMSO-d₆) : 1.77-1.89 (1H, m), 2.07-2.59 (3H, m), 3.14-3.25 (1H, m) , 3.24 (2H, t, J=8.6Hz), 3.39 (1H, d, J=13.6Hz), 3.43-3.51 (1H, m), 3.89 (1 H, d, J=13.6Hz), 4.06-4.09 (1H, m), 4.59 (2H, t, J=8.6Hz), 5.00-5.03 (1H, m ), 7.42-7.54 (3H, m), 7.73-7.79 (3H, m), 7.88-7.90 (1H, m), 7.93-7.98 (1H, d d, J=5.4Hz, 8.1Hz), 8.64-8.76 (2H, m), 9.10 (1H, d, J=1.89Hz), 10.37 (2H, b r), 10.59 (2H, br)
Values of elemental analysis (C₂₅H₂₇N₃O·2HCl)
Calculated value C: 65.50 H: 6.38 N: 9.17
Measured value C: 65.38 H: 6.41 N: 9.22

### (Example 9)

(2S,3S)-3-[(5-phenyl-2,3-dihydrobenzofuran-7-yl)methyl]amino-2-phenylpiperidine hydrochloride
m.p.: 197 to 203°C (dec.)
NMR(DMSO-d₆) : 1.77-1.87 (1H, m), 2.02-2.44 (3H, m), 3.13-3.25 (1H, m) , 3.21 (2H, t, J=8.6Hz), 3.44 (1H, d, J=13.5Hz), 3.45-3.52 (1H, m), 3. 84 (1 H, d, J=13. 5Hz), 3.95 (1H, m), 4.53 (2H, t, J=8.6Hz), 4.94 (1H, m), 7.26-7.3 3 (1H, m), 7.38-7.63 (10H, m), 7.71-7.75 (2H, m), 9.30 (2H, br), 10.26 (2H, b r)
Values of elemental analysis (C₁₆H₂₈N₂O·2HCl)
Calculated value C: 68.27 H: 6.61 N: 6.12
Measured value C: 68.40 H: 6.58 N: 6.16

### (Example 10)

(2S,3S)-3-[(5-(2-trifluoromethylphenyl)-2,3-dihydrobenzofuran-7-yl)methyl]amino-2-phenylpiperidine hydrochloride
m.p.: 149 to 157°C (dec.)
NMR (DMSO-d₆) : 1.75-1.85 (1H, m), 2.02-2.39 (3H, m), 3.10-3.22 (1H, m) , 3.19 (2H, t, J=8.9Hz), 3.44-3.51 (1H, m), 3.48 (1H, d, J=13.5Hz), 3.80 (1 H, d, J=13.5Hz), 3.89 (1H, m), 4. 54 (2H, t, J=8. 9Hz), 4.93 (1H, m), 7.11-7.1 4 (2H, m), 7.36-7.81 (4H, m), 7.54-7.62 (1H, m), 7.66-7.73 (3H, m), 7.77-7. 81 (1H, m), 9.25 (2H, br), 10.33 (2H, br)
Values of elemental analysis (C₂₇H₂₇F₃N₂O·2HCl)
Calculated value C: 61.72 H: 5.56 N: 5.33
Measured value C: 61.59 H: 5.60 N: 5.40

### (Example 11)

(2S,3S)-3-[(5-(3-trifluoromethylphenyl)-2,3-dihydrobenzofuran-7-yl)methyl]amino-2-phenylpiperidine hydrochloride
m.p.: 188 to 197°C (dec.)
NMR (DMSO-d₆) : 1.77-1.87 (1H, m), 2.02-2.43 (3H, m), 3.13-3.22 (1H, m) , 3.23 (2H, t, J=8.8Hz), 3.45-3.52 (1H, m), 3.45 (1H, d, J=13.5Hz), 3.88 (1 H, d, J=13.5Hz), 3.94 (1H, m), 4.55 (2H, t, J=8.8Hz), 4.94 (1H, m), 7.40-7. 53 (3H, m), 7.61-7.75 (6H, m), 7.91-7.95 (2H, m), 9.30 (2H, br), 10.21 (2H, b r)
Values of elemental analysis (C₂₇H₂₇F₃N₂O·2HCl)
Calculated value C: 61.72 H: 5.56 N: 5.33
Measured value C: 61.91 H: 5.59 N: 5.28

### (Example 12)

(2S,3S)-3-[(5-(4-trifluoromethylphenyl)-2,3-dihydrobenzofuran-7-yl)methyl]amino-2-phenylpiperidine hydrochloride
m.p.: 233 to 247°C (dec.)
NMR (DMSO-d₆) : 1.77-1.87 (1H, m), 2.03-2.44 (3H, m), 3.13-3.24 (1H, m) , 3.23 (2H, t, J=8.8Hz), 3.50 (1H, d, J=13. 6Hz), 3.45-3.52 (1H, m), 3. 86 (1 H, d, J=13.6Hz), 3.95 (1H, m), 4.55 (2H, t, J=8.8Hz), 4.95 (1H, m), 7.41-7.87 (11H, m), 9.30 (2H, br), 10.28 (2H, br)
Values of elemental analysis (C₂₇H₂₇F₃N₂O·2HCl)
Calculated value C: 61.72 H: 5.56 N: 5.33
Measured value C: 61.62 H: 5.54 N: 5.42

### (Example 13)

(2S, 3S)-3-[(5-(3,5-bis(trifluoromethyl)phenyl)-2,3-dihydrobenzofuran-7-yl)methyl]amino-2-phenylpiperidine hydrochloride
m.p.: 170 to 183°C (dec.)
NMR(DMSO-d₆) : 1.77-1.87 (1H, m), 2.03-2.46 (3H, m), 3.10-3.22 (1H, m) , 3.24 (2H, t, J=8.7Hz), 3.42-3.48 (1H, m), 3.44 (1H, d, J=13.6Hz), 3.91 (1H, d, J=13.6Hz), 3.93 (1H, m), 4.58 (2H, t, J=8.7Hz), 4.94 (1H, m), 7.39-7.51 (3 H, m), 7.71-7.78 (4H, m), 7.98 (1H, s), 8.29 (2H, s), 9.30 (2H, br), 10.22 (2H , br)
Values of elemental analysis (C₂₈H₂₆F₆N₂O·2HCl)
Calculated value C: 56.67 H: 4.76 N: 4.72
Measured value C: 56.84 H: 4.78 N: 4.77

### (Example 14)

(2S,3S)-3-[(5-(4-methoxyphenyl)-2,3-dihydrobenzofuran-7-yl)methyl]amino-2-phenylpiperidine hydrochloride
m.p.: 177 to 185°C (dec.)
NMR(DMSO-d₆) : 1.77-1.86 (1H, m), 2.03-2.42 (3H, m), 3.13-3.22 (1H, m) , 3.19 (2H, t, J=8.6Hz), 3.42 (1H, d, J=13.5Hz), 3.44-3.52 (1H, m), 3.79 (3H , s), 3.84 (1H, d, J=13.5Hz), 3.96 (1H, m), 4. 51 (2H, t, J=8.6Hz), 4.95 (1H, m) , 6.98 (2H, d, J=8.9Hz), 7.41-7.65 (5H, m), 7.54 (2H, d, J=8. 9Hz), 7.71-7.76 (2H, m), 9.30 (2H, br), 10.31 (2Hbr)
Values of elemental analysis (C₂₇H₃₀N₂O₂·2HCl)
Calculated value C: 66.53 H: 6.62 N: 5.75
Measured value C: 66.35 H: 6.60 N: 5.79

### (Example 15)

(2S, 3S)-3-[(5-(5-trifluoromethyl-1,2,4-oxadiazole-3-yl)-2,3-dihydrobenzofuran-7-yl)methyl]amino-2-phenylpiperidine hydrochloride
m.p.: 179 to 187°C (dec.)
NMR(DMSO-d₆) : 1.76-1.86 (1H, m), 2.01-2.39 (3H, m), 3.10-3.22 (1H, m) , 3.25 (2H, t, J=8.8Hz), 3.42-3.48 (1H, m), 3.52 (1H, d, J=13.5Hz), 3.84 (1H, m), 3.86 (1H, d, J=13.5Hz), 4.54-4.68 (2H, m), 4.89 (1H, m), 7.41-7.54 (3H, m ), 7.65-7.71 (2H, m) , 7.87-7.90 (2H,m), 9.20 (2H, br), 10.20 (2H, br)
Values of elemental analysis (C₂₃H₂₃F₃N₄O₂·2HCl)
Calculated value C: 53.39 H: 4.87 N: 10.83
Measured value C: 53.53 H: 4.91 N: 10.70

### (Example 16)

(2S,3S)-3-[(2,3,6,7-tetrahydrobenzo[1,2-b:4,5-b']difuran-4-yl)methyl]amino-2-phenylpiperidine hydrochloride
m.p.: 231 to 250°C (dec.)
NMR(DMSO-d₆) : 1.74-1.86 (1H, m), 2.04-2.38 (3H, m), 2.76-3.07 (4H, m) ,3.10-3.23 (1H, m), 3.26 (1H, d, J=13.2Hz), 3.40-3.50 (1H, m), 3.70 (1H, d, J=13.2Hz), 4.00 (1H, m), 4.37-4.47 (2H, t, J=8.5Hz), 4.96 (1H, m), 6.62 (1H, s), 7.42-7.55 (3H, m), 7.68-7.75 (2H, m), 9.00 (2H, br), 10.43 (2H, br)
Values of elemental analysis C₂₂H₂₆N₂O₂·2HCl)
Calculated value C: 62.41 H: 6.67 N: 6.62
Measured value C: 62.62 H: 6.71 N: 6.48

### (Test Examples)

In the following, a receptor binding test and a test of antiemetic action against cisplatin-induced vomiting on the compounds of this invention will be described.

### (Test Example 1: Receptor Binding Experiment)

As a sample, NK₁ TACHYKININ RECEPTOR, HUMAN(CHO) diluted with incubation buffer (20 mM HEPES/NaOH buffer containing 1 mM MgCl₂, 0.1% BSA: pH 7.4) was used (18.75 µg protein/ml). As test compounds, the compounds obtained in the Examples 3, 6, 7, 9, 12 and 16 were used. Twenty µl of each test compound which had been prepared by performing serial 5-fold dilutions to a final concentration of 5.12×10⁻¹² M to 1×10⁻⁵ M was added to each well of MultiScreen having been pretreated with 0.1% polyethylene imine. And 20 µl of CP-99,994 (final concentration of 1.5 µM) and 20 µl of distilled water were added to determine the amount of nonspecific binding and the amount of total binding, respectively. Further, 20 µl of [³H] substance P (final concentration of 0.2 nM) and 176 µl of the above sample (3.3 µg protein) were added and incubated at 27°C for 1 hour. After one hour of incubation, the incubated mixture was filtered with suction and washed with a wash buffer (50 mM Tris/HCl buffer: pH 7.4) four times. The membrane fitted to each well of the MultiScreen was completely dried and punched inside each vial, and 5 ml of cocktail was added to give a sample to be measured. The radioactivity of [³H] substance P in each measured sample was determined with a liquid scintillation counter. The amount of [³H] substance P bound to receptor was calculated with the measured radioactive count and a binding inhibition curve was obtained. And IC₅₀ of each test compound was calculated from the pseudo Hill plot analysis of the same data to give a Ki value. As a Kd value, 0.05 nM, which was obtained from a saturation test, was used. The results are shown in Table 1 below.

**Table 1**

| Test Compound (Example No.) | Ki Value (nM) |
|---|---|
| 3 | 0.18 |
| 6 | 0.35 |
| 7 | 0.35 |
| 9 | 0.41 |
| 12 | 1.01 |
| 16 | 1.53 |

As can be seen from the results shown in Table 1, it has been found that the piperidine derivatives of this invention provide a strong antagonistic action against substance P.

### (Test Example 2: Antiemetic Action against Cisplatin-Induced Vomiting)

A test was conducted using male ferrets (around 1.2 kg body weight) as follows. Vomiting was induced by intraperitoneal administration of 10 mg/kg of cisplatin to the ferrets in a volume of 3 ml/kg. As test compounds, the compounds obtained in the Examples 3, 9, 12, 15 and 16 were used. Each test compound was administered intravenously through radial skin at a dose of 3 mg/kg, and immediately after the administration, cisplatin was administered intraperitoneally. Then vomiting symptoms due to cisplatin was observed over six hours. The vomiting symptoms were observed in terms of 2 categories: retching characterized by rhythmic contract motion in abdomen and vomiting characterized by throwing up gastric contents or the like. The results are shown in Table 2 in terms of rates of inhibition to a control group using the number of times symptoms of vomiting occurred as an index.

**Table 2**

| Test Compound | Rate of Inhibition (%) | |
|---|---|---|
| (Example No.) | Retching | Vomiting |
| 3 | 59.1 | 61.0 |
| 9 | 68.4 | 59.3 |
| 12 | 88.4 | 91.1 |
| 15 | 63.2 | 66.8 |
| 16 | 58.7 | 62.6 |

As can be seen from the results shown in Table 2, it has been found that the piperidine derivatives of this invention provide a strong antiemetic action.

### Industrial Applicability

The piperidine derivatives of this invention provide a remarkable antagonistic action against substance P; accordingly, it is expected that they are used as remedies or prophylactics for various diseases mediated by substance P. Thus the piperidine derivatives of this invention are very useful in the pharmaceutical industry.

## Claims

1. Piperidine derivatives having the following general formula [I]: 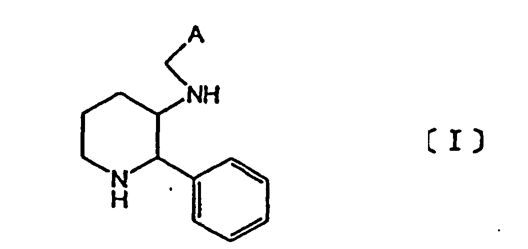 wherein A represents the following formula [A]: 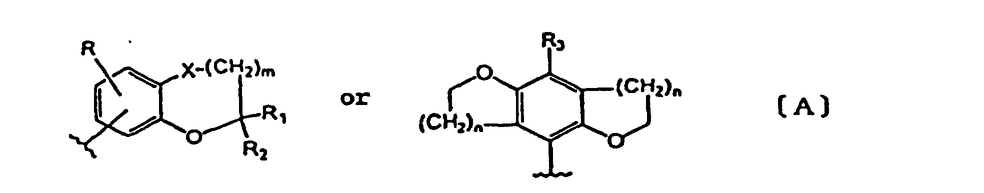 wherein
m is 0 or 1,
n is 1 or 2,
X represents CH₂, O or CH-CH₃,
R represents an unsubstituted or substituted phenyl group, the substituted phenyl group having 1 to 4 substituents of the
same or different kind, or an unsubstituted or substituted five-membered or six-membered heterocyclic group containing 1 to 4 heteroatoms selected from the group consisting of oxygen, nitrogen and sulphur atoms, the substituted heterocyclic group having 1 to 4 substituents of the same or different kind, said substituents being selected from a group consisting of halogen atoms, cyclic or noncyclic alkyl groups with 1 to 6 carbon atoms, which may contain 1 to 5 halogen atoms in arbitrary positions, cyclic or noncyclic alkoxy groups with 1 to 6 carbon atoms, which may contain 1 to 5 halogen atoms in arbitrary positions, and cyclic or noncyclic alkylthio groups with 1 to 6 carbon atoms, which may contain 1 to 5 halogen atoms in arbitrary positions,
R₁ and R₂ may be the same or different and represent a hydrogen atom or a cyclic or noncyclic alkyl group with 1 to 6 carbon atoms, respectively, and
R₃ represents a hydrogen atom or a cyclic or noncyclic alkyl group with 1 to 6 carbon atoms,
or pharmaceutically acceptable acid addition salts thereof.

2. Cis-piperidine derivatives according to claim 1 having the following general formula [Ia]: 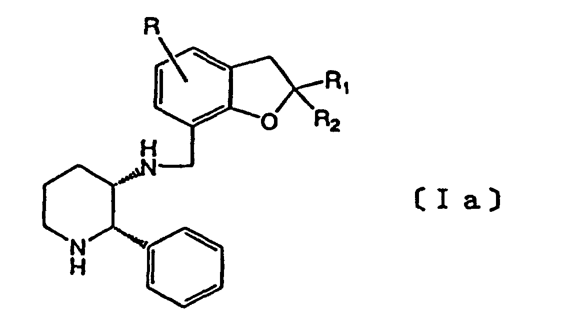 wherein
R represents an unsubstituted or substituted phenyl group or an unsubstituted.or substituted heterocyclic group as defined in claim 1,
R₁ and R₂ may be the same or different and represent a hydrogen atom or a cyclic or noncyclic alkyl group with 1 to 6 carbon atoms, respectively,
or pharmaceutically acceptable acid addition salts thereof.

3. Cis-piperidine derivatives according to claim 1 having the following general formula [Ib]: 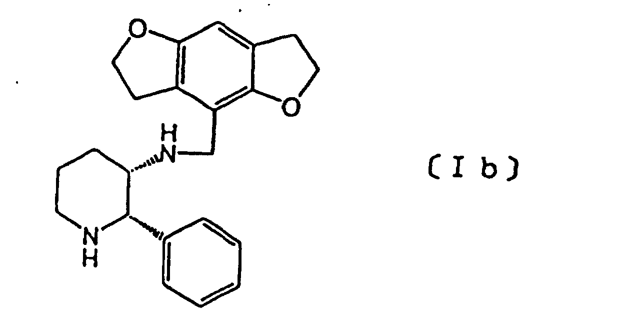 or pharmaceutically acceptable acid addition salts thereof.

4. Substance P antagonist compositions having as an effective component the piperidine derivatives or pharmaceutically acceptable acid addition salts thereof according to claim 1.

5. Substance P antagonist compositions having as an effective component the cis-piperidine derivatives or pharmaceutically acceptable acid addition salts thereof according to claim 2 or 3.

## Patentansprüche

1. Piperidinderivate mit der nachstehenden allgemeinen Formel [I] : 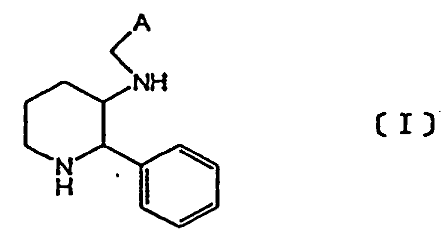 worin A die nachstehende Formel [A] repräsentiert: 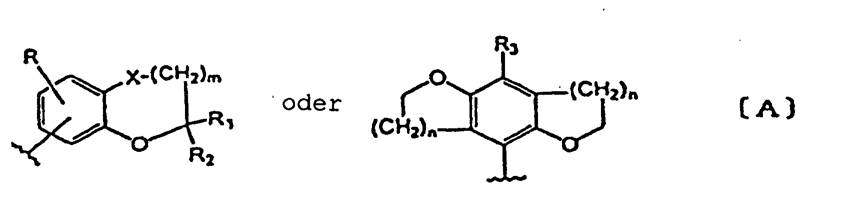 worin
m ist 0 oder 1,
n ist 1 oder 2,
X bedeutet CH₂, O oder CH-CH₃,
R bedeutet eine unsubstituierte oder substituierte Phenylgruppe, wobei die substituierte Phenylgruppe 1 bis 4 Substituenten aufweist, die gleich oder verschieden sind, oder eine unsubstituierte oder substituierte fünfgliedrige oder sechsgliedrige heterocyclische Gruppe, die 1 bis 4 Heteroatome, ausgewählt aus der Gruppe, bestehend aus Sauerstoff-, Stickstoff- und Schwefelatomen, enthält, wobei die substituierte heterocyclische Gruppe 1 bis 4 Substituenten aufweist, die gleich oder verschieden sind, wobei die Substituenten ausgewählt sind aus der Gruppe, bestehend aus Halogenatomen, cyclischen oder nichtcyclischen Alkylgruppen mit 1 bis 6 Kohlenstoffatomen, die an beliebigen Stellen 1 bis 5 Halogenatome enthalten können, cyclischen oder nichtcyclischen Alkoxygruppen mit 1 bis 6 Kohlenstoffatomen, die an beliebigen Stellen 1 bis 5 Halogenatome enthalten können, und cyclischen oder nichtcyclischen Alkylthiogruppen mit 1 bis 6 Kohlenstoffatomen, die an beliebigen Stellen 1 bis 5 Halogenatome enthalten können,
R₁ und R₂, die gleich oder verschieden sein können und ein Wasserstoffatom bzw. eine cyclische oder nichtcyclische Alkylgruppe mit 1 bis 6 Kohlenstoffatomen bedeuten, und
R₃ ein Wasserstoffatom oder eine cyclische oder nichtcyclische Alkylgruppe mit 1 bis 6 Kohlenstoffatomen bedeutet, oder pharmazeutisch akzeptable, durch Säureaddition erhaltene Salze davon.

2. Cis-Piperidinderivate gemäß Anspruch 1 mit der nachstehenden allgemeinen Formel [Ia]: 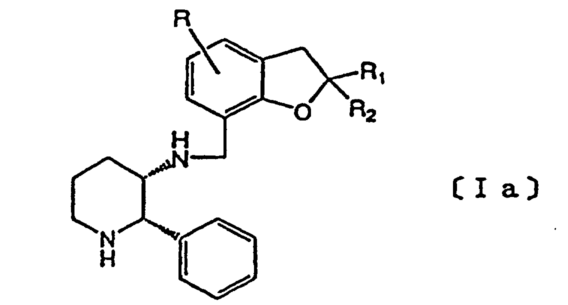 worin
R bedeutet eine unsubstituierte oder substituierte Phenylgruppe oder eine unsubstituierte oder substituierte heterocyclische Gruppe, wie in Anspruch 1 definiert,
R₁ und R₂ gleich oder verschieden sein können und ein Wasserstoffatom bzw. eine cyclische oder nichtcyclische Alkylgruppe mit 1 bis 6 Kohlenstoffatomen bedeuten,
oder pharmazeutisch akzeptable, durch Säureaddition erhaltene Salze davon.

3. Cis-Piperidinderivate gemäß Anspruch 1 mit der nachstehenden allgemeinen Fomel [Ib]: 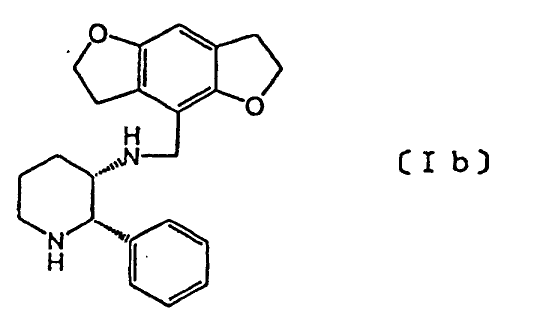 oder pharmazeutisch akzeptable, durch Säureaddition erhaltene Salze davon.

4. Zusammensetzungen von Substanz P-Antagonisten, die als wirksamen Bestandteil die Piperidinderivate oder pharmazeutisch akzeptable, durch Säureaddition erhaltene Salze davon gemäß Anspruch 1 aufweisen.

5. Zusammensetzungen von Substanz P-Antagonisten, die als wirksamen Bestandteil die Cis-Piperidinderivate oder pharmazeutisch akzeptable, durch Säureaddition erhaltene Salze davon gemäß einem der Ansprüche 2 oder 3 aufweisen.

## Revendications

1. Dérivés de pipéridine répondant à la formule générale [I] suivante : 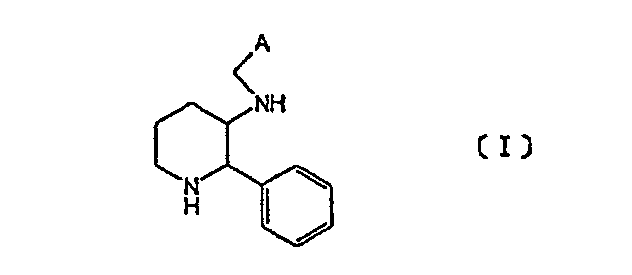 dans laquelle A représente la formule [A] suivante : 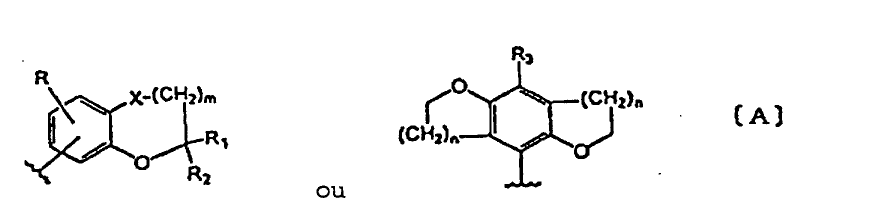 dans laquelle
m vaut 0 ou 1,
n vaut 1 ou 2,
X représente CH₂, O ou CH-CH₃,
R représente un groupe phényle non substitué ou substitué, le groupe phényle substitué comportant de 1 à 4 substituants de type identique ou différent, ou un groupe hétérocyclique à cinq chaînons ou à six chaînons non substitué ou substitué contenant de 1 à 4 hétéroatomes choisis dans le groupe constitué par les atomes d'oxygène, d'azote et de soufre, le groupe hétérocyclique substitué comportant de 1 à 4 substituants de type identique ou différent, lesdits substituants étant choisis dans un groupe constitué par les atomes d'halogène, les groupes alkyle cycliques ou non cycliques avec de 1 à 6 atomes de carbone, qui peuvent contenir de 1 à 5 atomes d'halogène en positions arbitraires, les groupes alcoxy cycliques ou non cycliques avec de 1 à 6 atomes de carbone, qui peuvent contenir de 1 à 5 atomes d'halogène en positions arbitraires, et les groupes alkylthio cycliques ou non cycliques avec de 1 à 6 atomes de carbone, qui peuvent contenir de 1 à 5 atomes d'halogène en positions arbitraires,
R₁ et R₂ peuvent être identiques ou différents et représentent un atome d'hydrogène ou un groupe alkyle cyclique ou non cyclique avec de 1 à 6 atomes de carbone, respectivement, et
R₃ représente un atome d'hydrogène ou un groupe alkyle cyclique ou non cyclique avec de 1 à 6 atomes de carbone,
ou les sels d'addition d'acide pharmaceutiquement acceptables de ceux-ci.

2. Dérivés de cis-pipéridine selon la revendication 1, répondant à la formule générale [Ia] suivante : 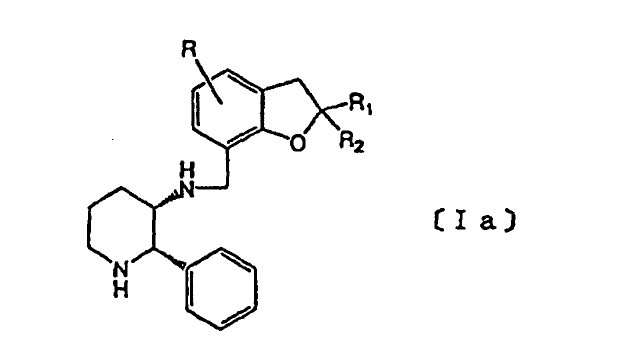 dans laquelle
R représente un groupe phényle non substitué ou substitué ou un groupe hétérocyclique non substitué ou substitué tel que défini dans la revendication 1,
R₁ et R₂ peuvent être identiques ou différents et représentent un atome d'hydrogène ou un groupe alkyle cyclique ou non cyclique avec de 1 à 6 atomes de carbone, respectivement,
ou les sels d'addition d'acide pharmaceutiquement acceptables de ceux-ci.

3. Dérivés de cis-pipéridine selon la revendication 1, répondant à la formule générale [Ib] suivante : 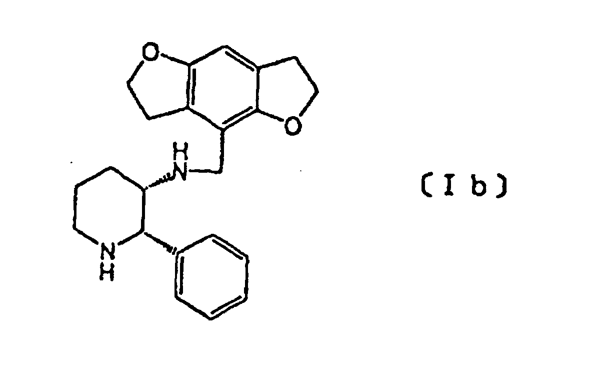 ou les sels d'addition d'acide pharmaceutiquement acceptables de ceux-ci.

4. Compositions antagonistes de la substance P ayant, en tant que composant efficace, les dérivés de pipéridine ou les sels d'addition d'acide pharmaceutiquement acceptables de ceux-ci selon la revendication 1.

5. Compositions antagonistes de la substance P ayant, en tant que composant efficace, les dérivés de cis-pipéridine ou les sels d'addition d'acide pharmaceutiquement acceptables de ceux-ci selon la revendication 2 ou 3.
